# EUROPEAN PATENT APPLICATION

(11) **EP 4 737 786 A1**
(43) Date of publication of application: **06.05.2026**
(21) Application number: 24210892.6
(22) Date of filing: 05.11.2024
(51) Int. Cl.: F16M 11/08, A61B 8/00, F16M 11/20, F16M 11/24, F16M 11/42, A61B 90/00

(54) **CART-BORNE ULTRASONIC IMAGING SYSTEM WITH IMPROVED LOCKING DEVICE**

(71) Applicant: ESAOTE S.p.A., 16152 Genova (IT)
(72) Inventor: Tedesco, Daniele, 16155 Genova (IT); Vigo, Valerio, 16149 Genova (IT)
(74) Representative: Bessi, Lorenzo

(57) **Abstract**

A cart-borne ultrasonic imaging system comprising:
a cart housing mounted on wheels;
a control panel or a support for a portable ultrasound unit;
an articulating arm assembly to which the control panel is connected for adjusting the position of the control panel, or the support for a portable ultrasound unit is connected for adjusting the position of the support for a portable ultrasound unit,
the articulating arm assembly including a first lower arm rotatably mounted on the cart and a second upper arm pivotably connected to the first lower arm and
rotatably connected to the control panel, or the support for a portable ultrasound device, wherein at least one of the arms includes a 4-bar linkage optionally comprising a main damping member, like a gas spring; and
a locking mechanism which acts to restrict motion of the articulating arm assembly. The locking mechanism comprises one or more electromagnetic elements controllable to exert a magnetic force on movable actuating elements comprising:
a friction mass coupled with a corresponding braking element acting to restrict rotation of the first lower arm with respect to the cart and/or of the control panel/the support for a portable ultrasound unit with respect to the second arm; and/or
a locking member kinematically coupled with main damping member acting to restrict the movement of such main damping member and thus of the 4-bar linkage,
wherein the electromagnetic elements are electrically actuated by a switch so that, upon activating the switch, the electromagnetic elements are energized to move synchronously the actuating elements in an unlocking position.

## Description

### FIELD OF THE INVENTION

The invention relates to the technical field of medical ultrasound imaging systems and, in particular, to lock devices, support arms thereof and ultrasound imaging systems using the same.

### STATE OF THE ART

Cart-borne ultrasound systems are now being designed for being more ergonomically comfortable for the user to operate. Particularly, control panels must be easily positioned (lowered, lifted or rotated) for comfort use during examinations. At the same time, once properly positioned, they must remain solidly in place and not move against the user's will.

Various methods have been proposed to optimize panel floating devices and their braking systems.

CN107835662 discloses a panel floating device comprising a translation mechanism, a floating device body and a rotation mechanism; the floating device body comprises a supporting seat and a top plate; the rotating mechanism is arranged on the top plate; the top plate is mounted on the support base by the translation mechanism and is translatable relative to the support base. This solution requires a motor to operate, hence is cumbersome, noisy and expensive. Furthermore, the use of a motorized system prevents the positioning of the panel in a single movement as height and rotation regulations are separated.

US9504447 discloses a control panel and a display of a cart-borne ultrasound system supported by a lift which can be controlled to allow the control panel and display to be raised, lowered and rotated. A pair of hydraulic struts are mounted to the four-bar linkage to support the weight of the control panel and display. When a control button on the handle of the control panel is depressed, the control panel and display can be freely raised, lowered and rotated. When the control button is released the pivot elbows and four-bar linkages are locked in their current positions. Each base plate has a circular arrangement of teeth which are engaged by a solenoid-controlled pin to lock the mechanism in its current rotated orientation. Hence, the system disclosed in this document has the disadvantage of not allowing the locking in any position but only in one of the allowed intermediate steps. To increase the number of the permitted positions, it is necessary to increase the number of teeth, but this means decreasing their thickness and consequently the mechanical resistance and thus increasing the risk of rupture. Furthermore, the complexity of the system makes the user experience unpleasant.

US8758246 discloses an ultrasound system which has a control panel adjustable in height. The system includes a power transmission device adjusting the height of the control panel, a contact detection unit detecting contact and generating a selection signal and a controller controlling the power transmission device according to the selection signal. This is advantageous, but the use of a control unit increases the cost and complexity of the solution.

Thus, a need continues to exist to have a system for locking/unlocking the positioning of the control panel of an ultrasound apparatus without obliged intermediate steps that limit the positioning or the use of expensive elements such as motors or control units.

### SUMMARY OF THE INVENTION

It is thus an object of embodiments herein to provide a cart-borne ultrasonic imaging system comprising:
a cart housing mounted on wheels;
a control panel or a support for a portable ultrasound unit;
an articulating arm assembly to which the control panel is connected for adjusting the position of the control panel, or the support for a portable ultrasound unit is connected for adjusting the position of the support for a portable ultrasound unit, the articulating arm assembly including a first lower arm rotatably mounted on the cart housing and a second upper arm pivotably connected to the first lower arm and rotatably connected to the control panel, or the support for a portable ultrasound device, wherein at least one of the arms includes a 4-bar linkage optionally including a main damping member such as a gas spring; and
a locking mechanism which acts to restrict motion of the articulating arm assembly.

The locking mechanism comprises one or more electromagnetic elements controllable to exert a magnetic force on movable actuating elements comprising:
a friction mass coupled with a corresponding braking element acting to restrict rotation of the first lower arm with respect to the cart and/or of the control panel/the support for a portable ultrasound unit with respect to the second arm; and/or
a locking member kinematically coupled with the main damping member acting to restrict the movement of the damping member and thus of the 4-bar linkage,
wherein the electromagnetic elements are electrically actuated by a switch so that, upon activating the switch, the electromagnetic elements are energized to move synchronously the actuating elements in an unlocking position.

This allows to realize a structure able to guarantee a wide range of positioning of the control panel, a simple and fast regulation and a synchronized lock/unlock of all the degree of freedom without the use of motorized systems or control units.

At least one electromagnetic element may be configured to move, when energized, the corresponding friction mass from a locking position, wherein such mass is in contact with a braking element integral with the first arm) or the second arm, to an unlocking position, wherein such mass does not interfere with such braking element to restrict rotation of the first lower arm with respect to the cart and/or of the control panel/the support for a portable ultrasound unit with respect to the second arm.

The locking mechanism may be advantageously configured to restrict the rotation of the lower arm with respect to the cart or of the control panel/support with respect to the upper arm on a horizontal plane, wherein the braking element is an element having, at least partially, the shape of a disk integral with the lower arm or the upper arm and the electromagnetic element is housed on the cart or the control panel/support to exert a magnetic force to move the friction mass in a direction transversal to the disk, particularly perpendicularly to the disk, to realize a magnetic-controllable disk brake.

If rotary bearings are used between the lower arm and the cart and/or between the control panel/support and the upper arm, the braking element may be an element having, at least partially, the shape of a disk integral with the part of the bearing associated with the lower arm or the upper arm.

In a preferred solution, the system comprises two electromagnetic elements and two corresponding braking elements to lock/unlock rotational movement of the first arm with respect to the cart and of the control panel/support with respect to the second arm and thus of control panel/support with respect to the cart.

According to embodiments herein, the system comprises:
a first electromagnetic element coupled with a corresponding braking element to lock/unlock rotational movement of the first arm with respect to the cart;
a second electromagnetic element coupled with a corresponding braking element to lock/unlock rotational movement of the control panel/support with respect to the second arm;
a third electromagnetic element coupled with the main damping member to lock/unlock the movement of the 4-bar linkage,
wherein the electromagnetic elements are actuated synchronously by the same switch so that, upon activating the switch, the full motion of the articulating arm assembly is allowed.

The switch may be selected from the group consisting in: a pedal located on the cart, a button located on a frontal handle of the control panel or of the support for the portable ultrasound device, a button located on the cart, a button located on the frame of a flat panel display electrically coupled to imaging electronics contained in the cart housing and/or in the portable ultrasound unit, a pedal used to brake the wheels of the cart or combination thereof.

Elastic elements may be provided to keep the friction mass(es) pushed onto the braking element(s) when the electromagnetic element(s) are not actuated by the switch.

In an embodiment, an O-ring, a gasket, a rubber element or the like is placed on the surface of the friction element(s) facing the braking element(s) to increase friction.

In an embodiment, one or more damping members, further to the main damping element, may be used. This allows to improve the self-balancing of the pantograph to avoid collapsing of the control panel once unlocked and to reduce the effort needed for the motion, thanks to an adequate force analysis.

One or more of the damping members may be advantageously lockable/unlockable by the same or a different electromagnetic element locking/unlocking the main damping member.

The system may comprise an indicator providing information on the status of the locking mechanism, particularly with a luminous indication in green/red or any other suitable colour, for example surrounding the button actuating the electromagnet(s).

According to an improvement, the system may comprise a mechanical lock to secure the magnetic braking during transportation, such lock comprising a first lever moving a slidable pin into a corresponding hole of the rotary bearing(s) when secure locking is to be achieved.

In an embodiment, the mechanical lock comprises a pin holder and an elastic element, wherein the pin is installed inside a cavity of the pin holder against the force of the elastic element so that, when the lever is activated, the pin slides consequently inside the pin holder to protrude from the pin holder to engage a hole in a bracket integral with the corresponding bearing, locking its rotation.

The first lever of the mechanical lock may be advantageously located on the cart and/or on the control panel/support for a portable ultrasound device in a position accessible by a user. If the mechanical lock is to be positioned in a not easily accessible position, a remote lever may be used. This remote lever may be placed in an easily accessible location for a user and connected with the mechanical lock through a wiring cable to transmit motion from the lever to the slidable pin.

In an embodiment, the ultrasound system comprises a control panel supported by a floating device comprising a four-bar linkage preferably equipped with a gas spring. The four-bar linkage comprises a first bearing on the bottom, to allow the rotation around the vertical axis of the floating device relative to the main body, and a second bearing on the top, to allow the rotation around the vertical axis of the control panel relative to the pantograph. The floating device can be raised or lowered, allowing the "up and down" and "back and forth" movements.

Each bearing is integral with a shaped metallic bracket. The shape of the bracket is a complete disk, if the desired rotation is 360°, or part of a disk if the desired rotation is correspondingly reduced.

A first electromagnet, located on the main body, is in contact with the bracket of the bottom bearing, while a second electromagnet, located on the control panel, is in contact with the bracket of the top bearing.

The frictional force between each electromagnet and the corresponding bracket prevents the rotations around the vertical axis.

The four-bar linkage may also comprise a solenoid, integral with the internal structure, and with a rod normally pulled out. The rod between a linking mechanism drives the piston rod of the gas spring, so normally the gas spring is locked.

When the position of the control panel needs to be adjusted, providing an input (e.g. pushing a button) to an ON/OFF switch, an electric signal is generated. This electric signal energizes the electromagnets, that increase their distance from the corresponding brackets, and the solenoid, that retracts its rod.

The bearings to which the brackets are integral can now rotate, while the gas can move between the chambers of the gas spring and consequently the pantograph can be lowered or raised.

As the electromagnets and the solenoid are energized, the control panel can rotate relative to the floating device, the floating device can be lifted or lowered, the floating device can rotate relative to the main body, or any combination of the previous movements is possible.

Once the need is over, the change of status of the switch ON/OFF returns all the parts (electromagnets and the rod of the solenoid) in their normal configuration ("locked status"), keeping the control panel at the desired position.

In a specific configuration, all the movements can be unlocked by pushing a button, for example, located on the front handle of the control panel.

In another embodiment, the button is a foot actuated button located on the wheel assembly.

During transportation, for safety reasons, a further mechanical lock can be used, in particular for assuring a safe lock of rotations. In fact, during transportation vibrations and impacts may appear, causing forces that may win the frictional force and cause a safety issue. The mechanical lock may advantageously comprise a lever that, directly or by a wire cable like a Bowden cable, drives a pin that engages or disengages a hole in a bracket integral with the bearings or directly in the bearings, locking or unlocking the rotations.

The mechanical lock may be associated to each electromagnet or just to one depending on the required level of safety.

Further improvements of the invention will form the subject of the dependent claims.

### BRIEF DESCRIPTION OF THE DRAWINGS

The characteristics of the invention and the advantages derived therefrom will be more apparent from the following description of non-limiting embodiments, illustrated in the annexed drawings, in which:
Fig. 1 shows a perspective view of a conventional ultrasound apparatus according to the state of the art.
Fig. 2 is a perspective view of a pantograph movement system according to embodiments herein that can be used with the apparatus of Fig. 1.
Fig. 3 is a side view of the movement system of Fig. 2.
Fig. 4 is a side view of the movement system of Fig. 2 with a partial cross section showing the mechanism locking the movement of the articulating arms of the pantograph according to embodiments herein.
Fig 5 is a side view of the movement system of Fig. 2 with partial cross section showing the mechanism locking the rotation of the pantograph with respect to the cart according to embodiments herein.
Fig. 5a is an enlarged view of the partial cross section of Fig. 5.
Fig. 6 is a flowchart of the process for adjusting the control panel position according to an embodiment herein.
Fig. 7a and 7b are enlarged views of the locking mechanism inside the pantograph of Fig. 4 in two operating positions according to embodiments herein.
Fig 8 is an enlarged view of the rotational locking mechanism of Fig. 5 in two operating positions (unlock/lock status).
Fig. 9 is a top view of an apparatus including mechanical locks according to embodiments herein to be used during transportation.
Fig. 10 is a perspective view of a mechanical lock according to an embodiment.
Fig. 11 is a detailed view of the lever of the mechanical lock where the ergonomics of use can be appreciated.
Fig. 12 is an exploded view of the mechanical lock of Fig. 10.
Fig. 13a is an enlarged view of the mechanical lock of Fig. 10 according to an embodiment where the plastic cover surrounding the mechanical lock is tamp graphed with padlock symbols.
Fig. 13b is an enlarged view of the mechanical lock of Fig. 10 according to a second embodiment where the lever is printed with padlock symbols.
Fig. 14a and 14b are cross sections of the mechanical lock of Fig. 10, respectively, in the unlock and lock status.
Fig. 15 is a view of the articulated arm with a remote lever connected with a Bowden cable to the mechanical lock.
Fig.16 is an exploded view of the embodiment of a mechanical lock with a Bowden cable.
Fig. 17 shows the usability of the apparatus according to the embodiments herein during transportation.
Fig. 18a and 18b are perspective views of an ultrasound apparatus according to a further embodiment where the release button is a pedal located on the wheel assembly.
Fig. 19 is a partial perspective view of an ultrasound apparatus according to a further embodiment where the release button is surrounded by a strip of led indicating the status of the button.
Fig. 20 is a perspective view of a trolley for portable apparatus with the pantograph and the lock system according to embodiments herein.
Fig. 21 and 22 show a perspective view of a further embodiment where the release button is located on the handle of the monitor and the braking system acts on the monitor arm.
Fig. 23 is a perspective view of a trolley for LCD touch apparatus with pantograph and lock system according to embodiments herein.

### DETAILED DESCRIPTION OF A PREFERRED EMBODIMENT

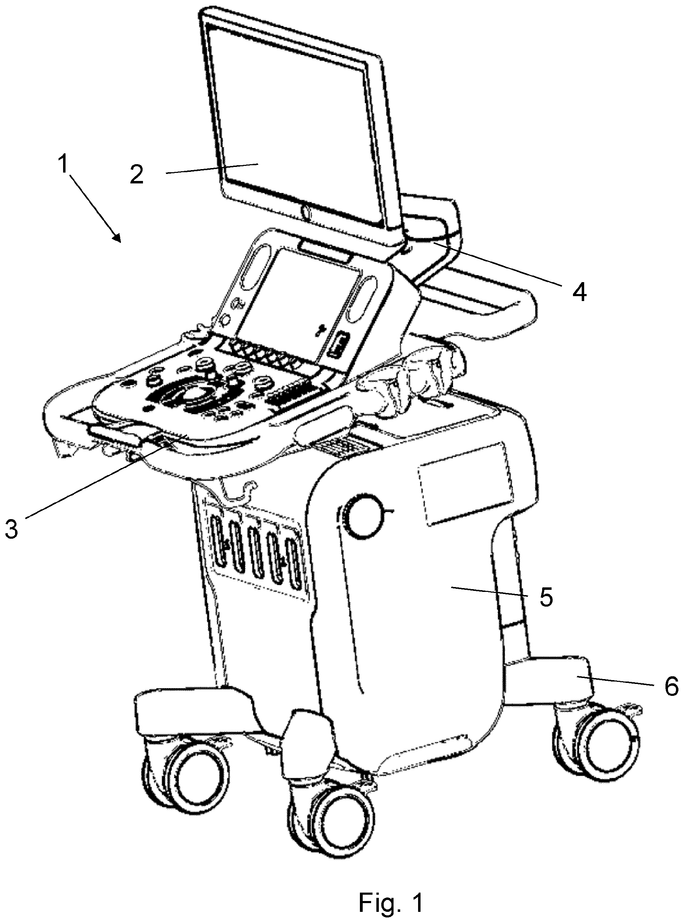

Referring to Fig. 1, a conventional ultrasound apparatus 1 includes a cart housing 5, a control panel 3, a monitor arm 4 and a display 2. The control panel 3 is mounted on the main body 5, in turn mounted on the wheel assembly 6.

The display 2 shows the results of the examination, the monitor arm 4 is used to support and, eventually, position the display 2, the control panel 3 is used to set operation parameters and/or commands by pressing buttons or acting on the touch screen. The control panel 3 has a handle 30 to be grasped for positioning and holding up probe holders, gel holder, gel warmer, cable hooks or the like.

The control panel 3 is typically moved by using an articulating arm assembly 9, also referred in the present disclosure as pantograph or floating device. The articulating arm assembly 9 includes a first lower arm 109 rotatably mounted on the cart 5 and a second upper arm 209 pivotably connected to the first lower arm 109 and rotatably connected to the control panel 3. The assembly 9 typically comprises a four-bar linkage 209 with a gas spring 97 for damping and supporting purposes. It may also comprise an internal cable guide to avoid the monitor cables to be damaged during the pantograph movement and plastic covers in order to protect user's hands, for aesthetics and for protecting the inside mechanism from dirt.

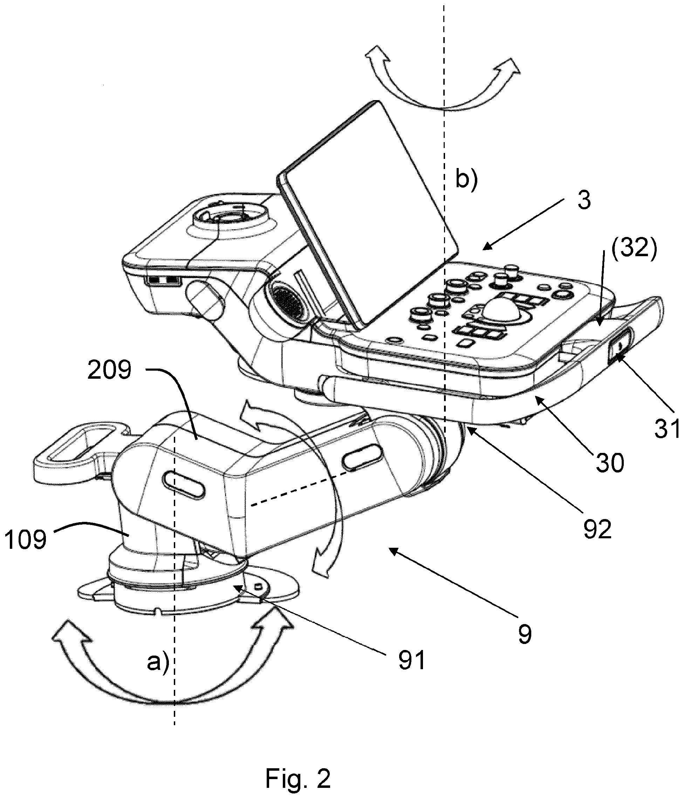

The wheel assembly 6 comprises the wheels for the movement of the full apparatus 1 and their braking system. As shown in Fig. 2, multiple movements are possible at the same time: the pantograph 9 can be lowered or lifted realizing at the same time the up-and-down and the back-and-forth movements (intrinsically linked to each other) and can rotate around the vertical axis a) relative to the cart housing 5 through a first bearing 91. Through a second bearing 92, the control panel 3 can rotate around the vertical axis b) with respect to the pantograph 9. In this way, the control panel 3 is adjustable to accommodate a certain range of operator heights and operating positions.

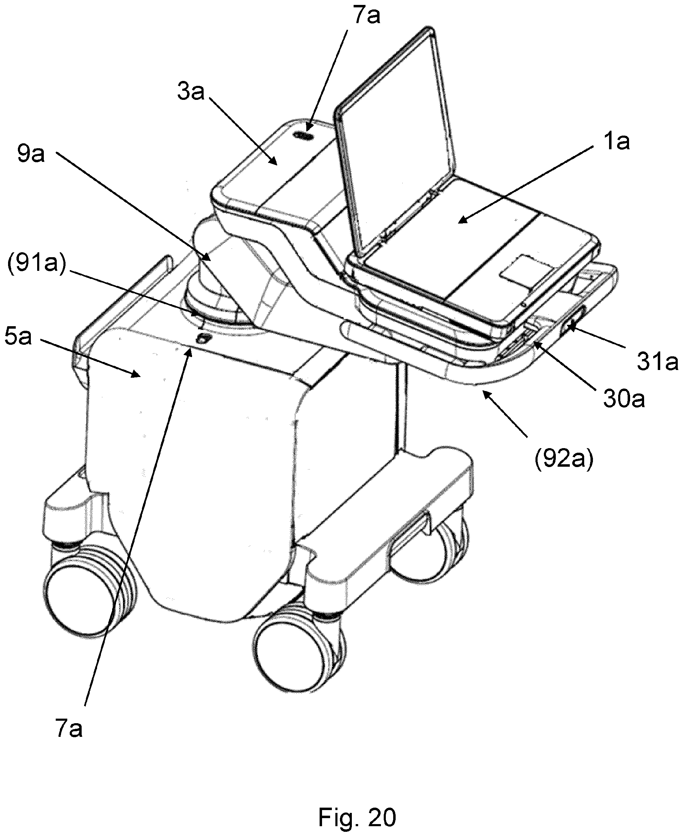

In this configuration, the main electronic of the ultrasound system is contained in the cart housing 5. When portable devices are used, the main electronic is contained in a portable ultrasound device 1a placed on a support 3a replacing the control panel 3 as shown in Fig. 20. Hereinafter, the term control panel is to be seen to include also the support for an ultrasound portable unit being the articulating structure the same.

As it will be disclosed in detail below, the idea at the base of the invention is to lock the rotation of the pantograph 9 relative to the cart housing 5, and/or the rotation of the control panel 3 relative to the pantograph 9, by using the frictional force induced by a mass moved by an electromagnetic element that can be activated/deactivated by a user, for example using a button 31 placed on the handle 30 used to grasp the control panel 3.

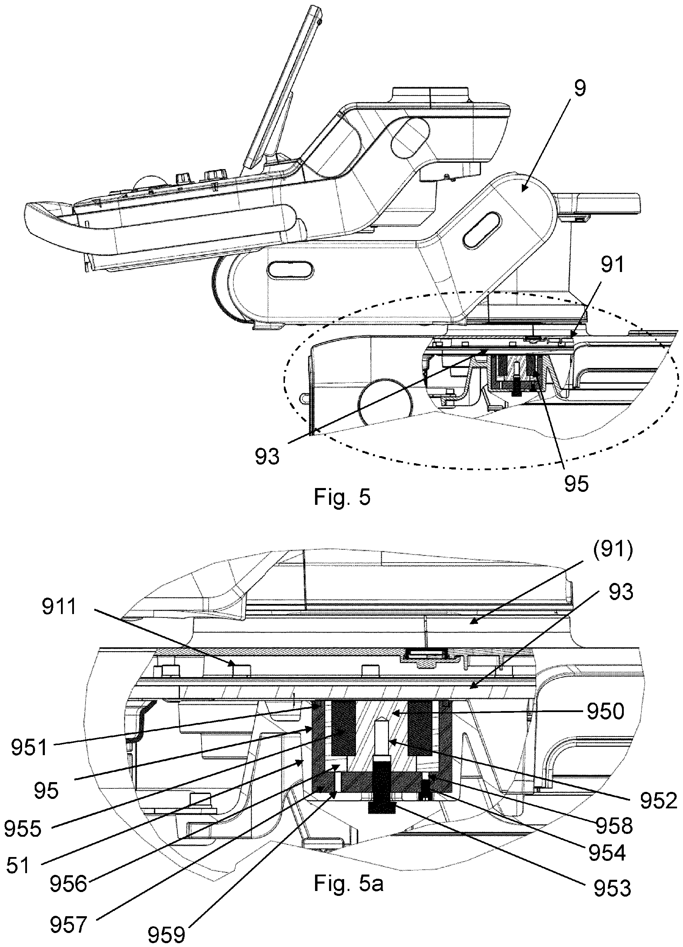

As best shown in Fig. 5a, the "normally closed" electromagnetic element 95, integral with the cart housing 5, move a mass 950 towards and backwards the bracket 93 integral with bearing 91 to realise a sort of magnetically controlled brake disk.

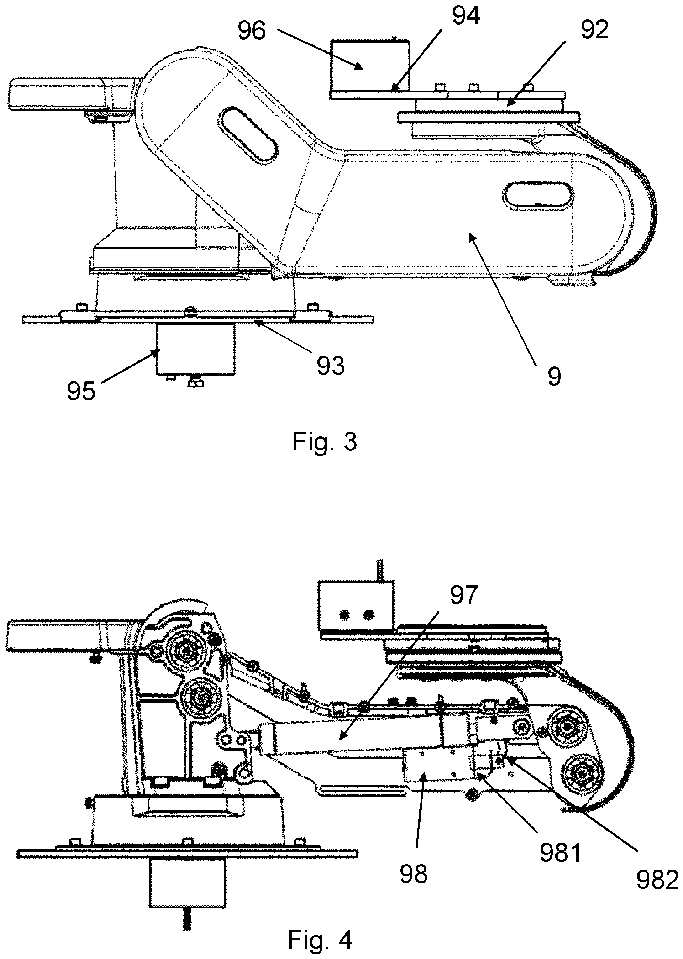

In the same way, the rotation of the control panel 3 relative to the pantograph 9 may be locked by the frictional force induced on the bracket 94 integral with bearing 92 by the mass moved by the electromagnetic element 96 integral with the control panel 3 (see Fig. 3).

In an embodiment, the electromagnetic element 95, 96 comprises (as shown in Fig. 5a) a coil 955 whose wire is wrapped around a core 950 of ferromagnetic material like iron. The coil 955 and the core 950 are contained in a pot-like casing 957 and are sealed with synthetic resin 956. This assembly could be placed in a cylindrical casing 51 in the main body 5 or in the control panel 3. The electromagnetic element 95 has conveniently a threaded hole 952 to receive a first screw 953 and the housing 957 has a threaded hole 958 to receive a second screw 954 and a hole 959 to let the wires pass through. The screw 954 is useful to prevent the rotation of the electromagnetic element 95 around the vertical axis, while the screw 953 could be used to pull down manually the electromagnetic element 95 in case of need using its head as a knob. It could also be advantageously present a thermal switch (not shown) that breaks the electric circuit in case of overheating.

In the described example, the mass moved by the electromagnetic element 95, 96 is the mass of the core 950 and of the housing 957 integral with such core. This is not to be considered a limiting feature as, in general, any device that is responsible for imparting a magnetic force allowing a braking mass to move away from, and/or towards, the brackets could be used for the purpose. Such device may be, for example, a coil with a magnetic core acting as a piston on a braking mass kinematically linked to an extremity.

For safety reasons, the magnetic force is preferably in the direction of attraction of the mass away from the brackets to maintain the locking device in the locked position when the electromagnetic element is not energized. However, the magnetic force could also act on the opposing direction, i.e. by pushing the mass towards the disk brake. In this case the braking action is achieved by powering, rather than de-energizing, the electromagnetic element. A bidirectional movement of the mass under the action of the electromagnetic element could equally be envisaged.

The frictional force acted by one or both the electromagnetic elements 95, 96 may be increased thanks to a rubber gasket 951 positioned on the surface of the frictional mass facing the corresponding bracket 93, 94 as shown in Fig. 5a. It should be noted that using two electromagnetic elements 95, 96 of the same type leads to greater simplicity and cost savings although this is not essential as only one electromagnetic element may be used or two electromagnetic elements of different type as well.

As shown in Fig. 4, in an embodiment, also the lift of the pantograph 9 may be locked. This is achieved by a further lock that may also exist independently of the rotational locks described. A solenoid 98 is fixed on the internal structure of the parallelogram assembly and in this configuration the metallic rod 981 is all pulled out. The rod 981 between a linking mechanism 982 (e.g. a hinged lever, screwed to the rod 981) locks the piston rod 971 of the lockable gas spring 97, so in this configuration the gas spring 97 is locked. In detail, the gas spring 97 comprises a valve in the piston that allows to lock the stroke in any position. When the lock/unlock pin on the top of the piston rod 971 is pressed the valve opens and the gas spring 97 can be positioned into the desired position. Releasing the pin the stroke will be locked. So, this system advantageously allows to lock the stroke of the gas spring and consequently the lift of the pantograph 9 in any position. The pantograph locking could also exist independently from the rotation locking of the first arm and/or of the control panel disclosed above.

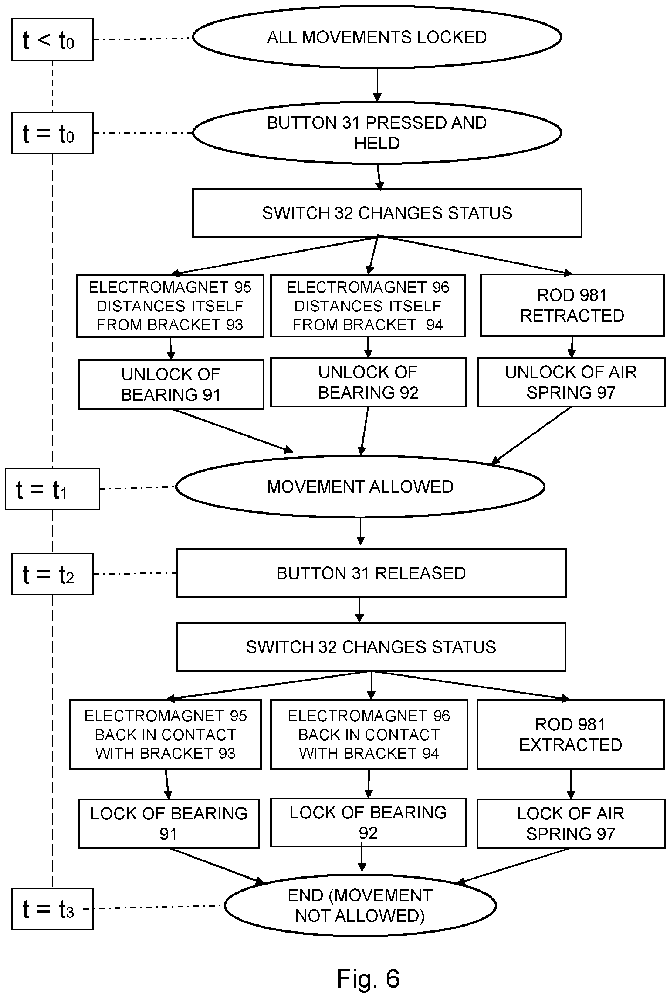

Referring now to the flow chart of Fig. 6, starting from an initial step where all the positions and the movements are locked, when there is a need to change the position of the control panel 3, the button 31, for example, located on the frontal handle 30 can be pushed. The movement of the button 31 makes the switch ON/OFF 32 change status. The electrical signal generated acts simultaneously on the electromagnet 95, electromagnet 96 and solenoid 98, consequently causing the distancing of the electromagnets 95, 96 from the corresponding brackets 93, 94 and the retraction of the rod 981 (due to the magnetic field generated) thus unlocking the bearing 91, bearing 92 and gas spring 97.

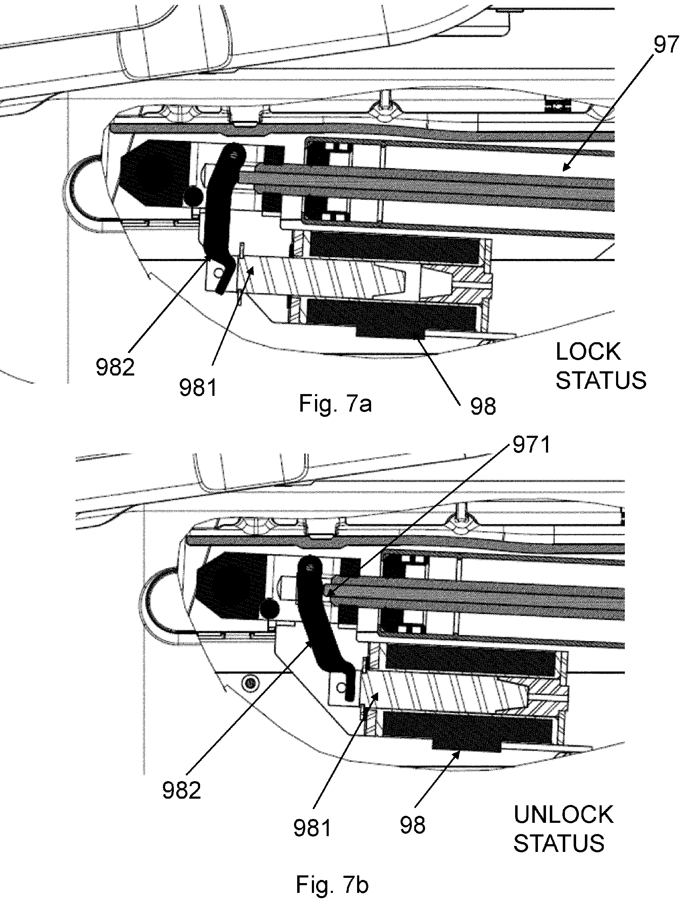

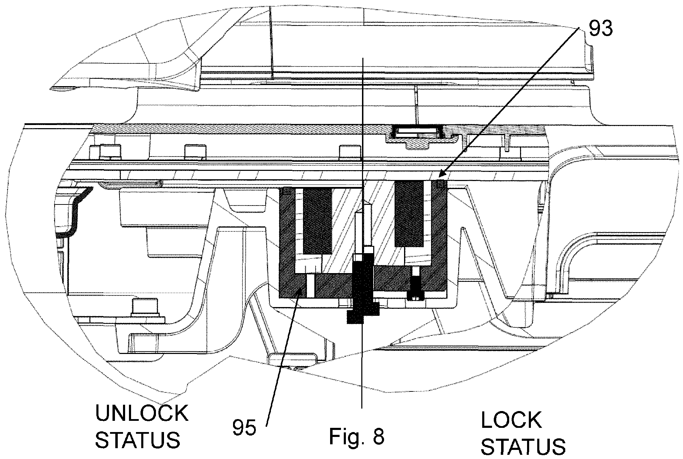

These movements are illustrated in Fig 7a-7b and 8: Fig 7a shows the rod 981 in the locked status, while Fig. 7b shows the rotation of the mechanism 982 due to the retraction of the rod 981 in the unlocked status that causes the retraction of piston rod 971 of the gas spring 97.

In Fig. 8, on the right, the electromagnet 95 in the normal status (locked) is visible: the electromagnet 95 has the upper face in contact with the bracket 93; on the left, the electromagnet 95 in the unlocked status is visible: a gap with the bracket 93 is present.

With button 31 pressed, the user can thus rotate and lift the control panel 3 almost freely in the 3D space. Once reached the desired position, the button 31 can be released, causing the switch 32 to change status and again the lock state of electromagnet 95, electromagnet 96 and solenoid 98 and consequently the complete lock of the control panel 3. It should be noted that with a single electric button all the three movements may be unlocked synchronously without the need of a control unit. The switch 32 could equally control only one among electromagnet 95, electromagnet 96 and solenoid 98 or any combination of two of these components.

Choosing normally closed electromagnets allows to save energy and prevent potential burnout. Moreover, in case of lack of energy, it is safer to have the movements locked rather than unlocked.

Springs (not shown in the figures) may be present to produce elastic-restoring force that may counteract the residual magnetic force so that when electromagnets 95 or 96 are not activated, the associated masses interfere with the respective brackets.

A second gas spring may be advantageously used to help balancing the weight of the control panel 3 so that when the lift of the pantograph 9 is unlocked, the control panel 3 doesn't collapse and the effort made by the user for the positioning is reduced.

This is achieved by an accurate study of the forces acting on the pantograph 9 in different configurations and the consequent choice of the more appropriate springs. For example, the force of the gas spring 97 and the gravitational force can be balanced by acting on the position of the centre of gravity and the position of the lever arms with respect to the swivel point.

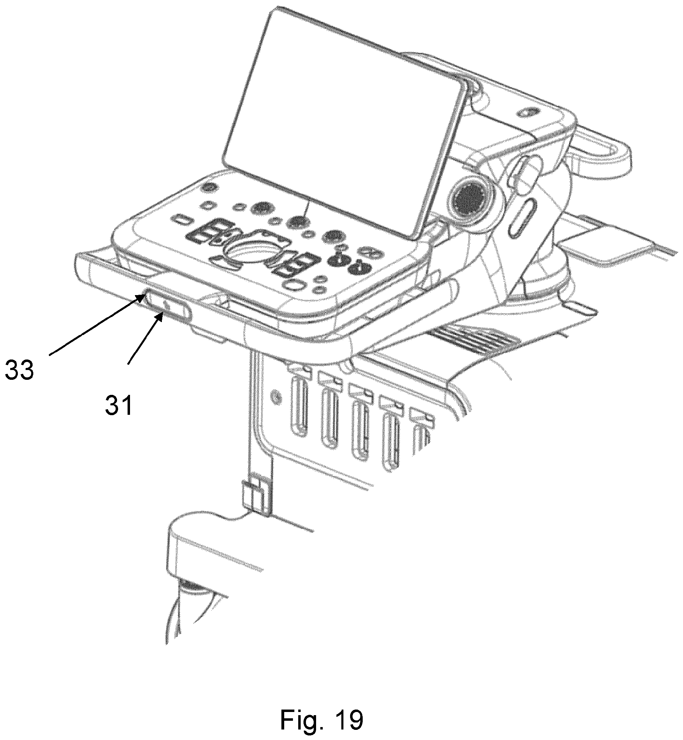

For a better usability, the symbol of an open padlock can be embossed on the button 31 (as shown in Fig. 2 and 19) to make evident its function.

During transportation, for safety reasons, a further mechanical lock 7 can be used, preferably, but not necessarily, one for each electromagnet 95, 96 to assure safe lock against accidental rotations. In fact, during transportation, vibrations and impacts may appear, causing additional forces that may overcome the frictional force, causing unwanted movements of the pantograph 9 and possible collisions or damages.

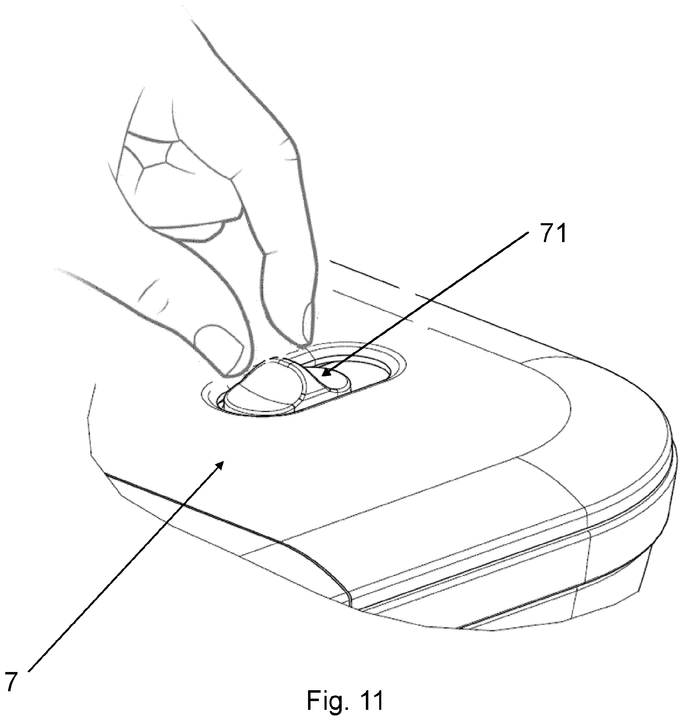

With reference to 10, the mechanical lock 7 essentially comprises a first lever 71 moving a slidable pin 77 into a corresponding hole of the rotary bearing 91, 92 when secure locking is to be achieved. The lever 71 can be advantageously designed to be ergonomically manipulated; for instance, it could have concave nests conforming to finger shape for a comfortable grip, as visible in Fig. 11. Pin 77 advantageously has a cylindrical shape with at one end two opposite facets 771 with two through holes 772 and on the other end a bigger diameter 773 acting as a bump stop for the spring 73.

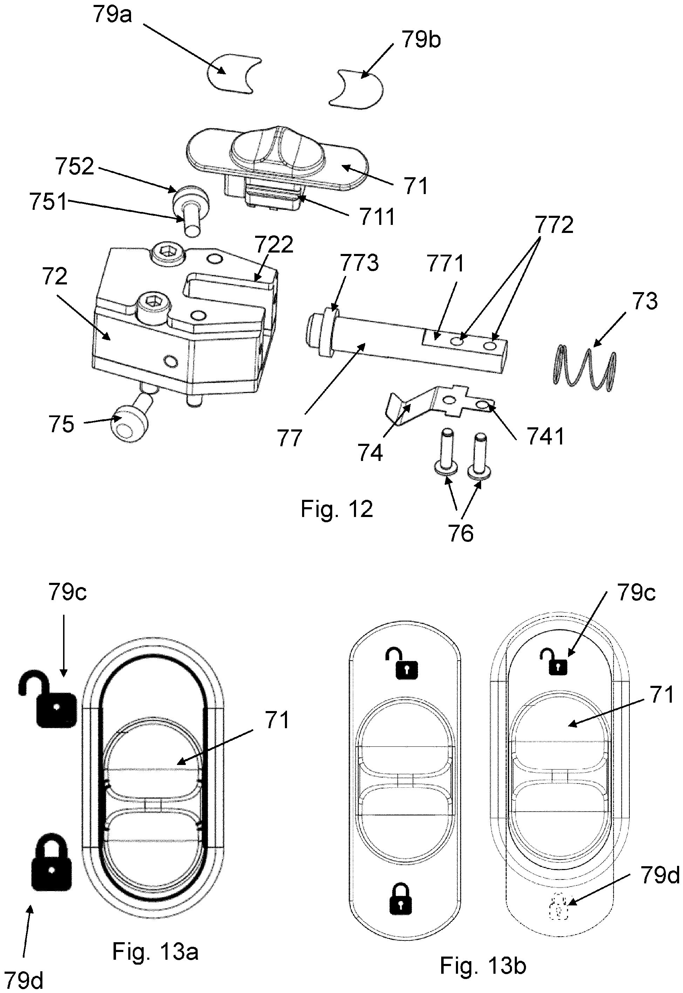

In the embodiment shown in Fig. 12, the mechanical lock 7 further comprises a pin holder 72, two springs 73, 74, two bumpers 75 and two screws 76. The spring 73 is a cylindrical spring made by a wire coil, while the spring 74 is a thin bent sheet of metal having two holes 741 for the fixing. The pin 77 is installed inside the hole 721 of the pin holder 72 against the spring 73. The pin 77, the lever 71 and the spring 74 are made integral with each other by the screws 76. The lever 71 can slide along the pin holder 72 thanks to the coupling between the grooves 711 in the lever 71 and the corresponding protrusion in the pin holder 72.

In this way, when the lever 71 is pushed, the pin 77 slides consequently and of the same quantity "X" inside the pin holder 72 and the spring 74 gets compressed.

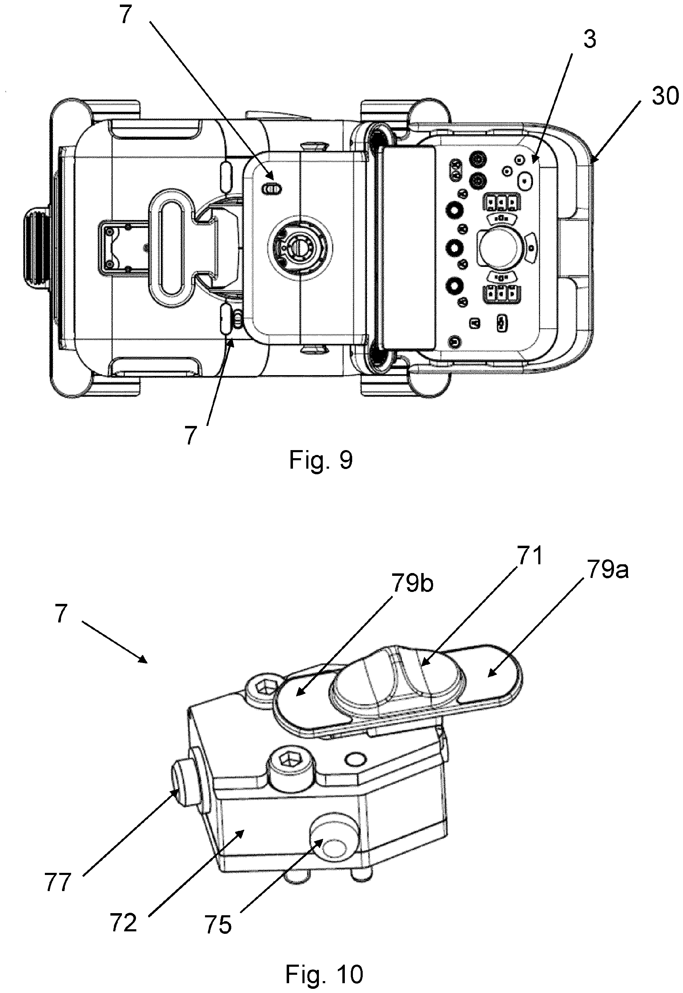

The mechanical lock 7 can be installed on the main body 5 or on the control panel 3 as exemplary shown in Fig. 9.

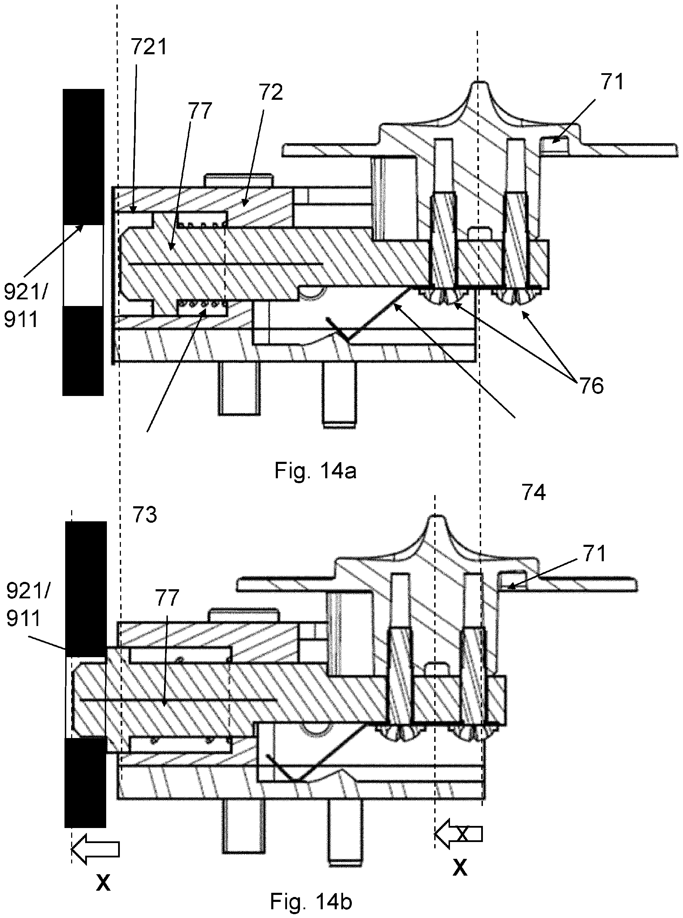

As shown in Fig. 14, pin 77 in the locked configuration engages a hole in a bracket 921, 911 integral with the corresponding bearing 92, 91, locking its rotation.

Pulling back the lever 71, the pin 77 disengages the hole, letting the bearing 92 or 91 free to rotate. At the same time, the spring 73 gets compressed and the spring 74 extends.

The alternative compression of the springs 73 and 74 helps the movements of the lever thanks to their elastic return.

The bumpers 75 located on the side of the pin holder 72 act as bump stop for the bracket 92, 91 in case of collisions. In Fig. 10 It can be noted that an advantageous bumper comprises a threaded stem 751 for the fixing and the rubber head 752 for the damping function.

To help the user to identify if the lock is active or not, a red label 79a and a green label 79b can be applied on lever 71 such as, when the lock is active, the colour visible in the slot in the cover of the main body 5 or control panel 3 is the red one, while, when the lock is unactive, the green colour is the one visible. Alternatively, symbols of an open padlock 79c and of a closed one 79d can be tamp graphed (pad printed) on the plastic cover of the main body 5 or control panel 3 as shown in Fig. 13a or directly on the lever 71 as shown in Fig.13b, where only one symbol is visible at a time and the other one is covered by the plastic cover of the main body 5 or control panel 3.

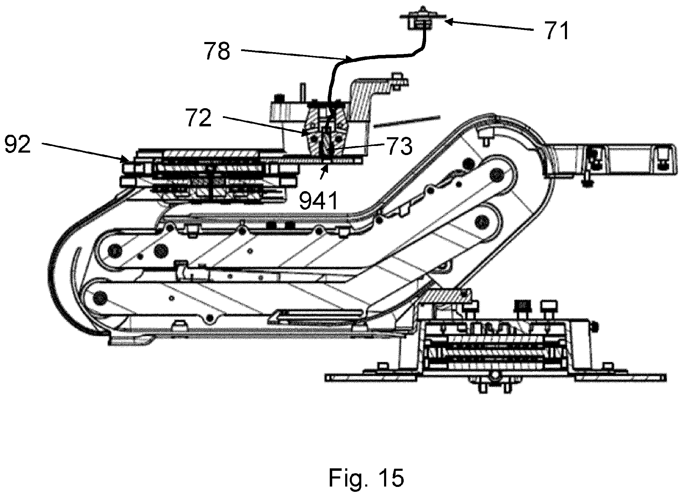

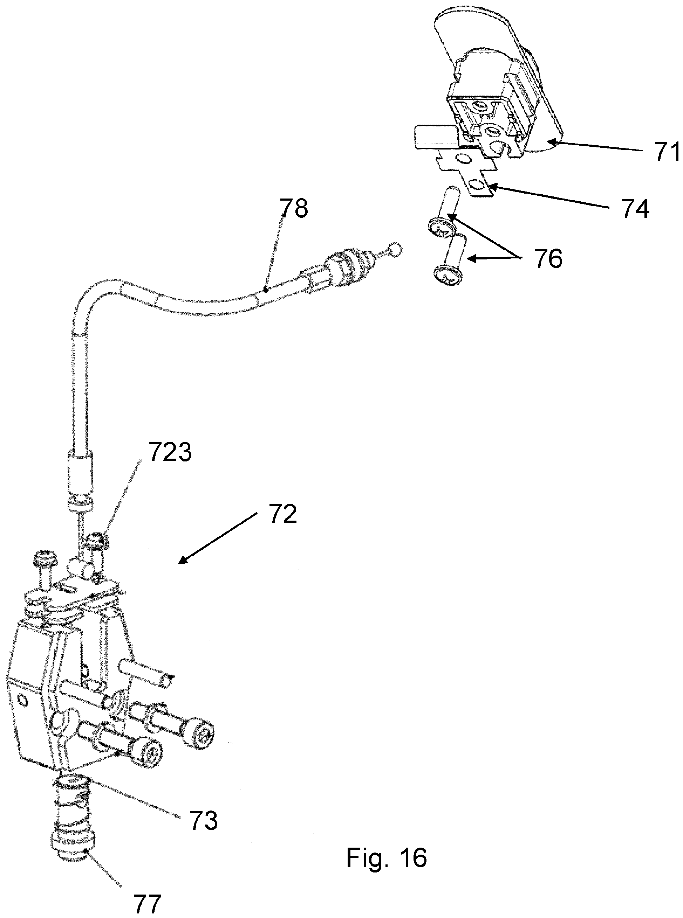

Referring to figures 15 and 16, in another embodiment, the mechanical lock 7 comprises a lever 71, a pin 77, a pin holder 72, two springs 73, 74, two bumpers 75, two screws 76, two sheet metal pieces 723 and a Bowden cable 78.

Pushing the lever 71, that slides along specific protrusions realized in the structure of the control panel 3, the Bowden cable 78 tenses up and transmit the motion to the pin 77 that engages the hole 941 in the bracket 94. Viceversa, pulling back the lever 71, the pin 77 disengages the hole 941 in the bracket 94. As the bracket 94 is integral with the bearing 92, this one is locked. The pin 77 and the lever 71, in this case, are shaped to receive each the respective end of the Bowden cable 78. The springs 73, 74 and the bumpers 75 work as previously described. The sheet metal pieces 723 are screwed to the pin holder 72 and have the function of guiding the Bowden cable 78.

This solution is useful for positioning the lever 71 in a location more easily accessible by a user in case of need.

The same type of mechanical lock 7 can be used for one or both the bearings 91, 92 depending on the design requirements.

It should be noted that providing only one hole on the bracket 93, 94 determines only one locking position, if necessary, multiple holes can be realized to have different locking positions: one position could be used, for instance, for the transportation from the manufacturer to the end user where the control panel 3 is 180 degrees rotated (for a low volume package), another position could be used for the transportation by foot from a hospital ward to another one where the control panel 3 is located in a straight-ahead position, particularly in the absence of a handle on the back.

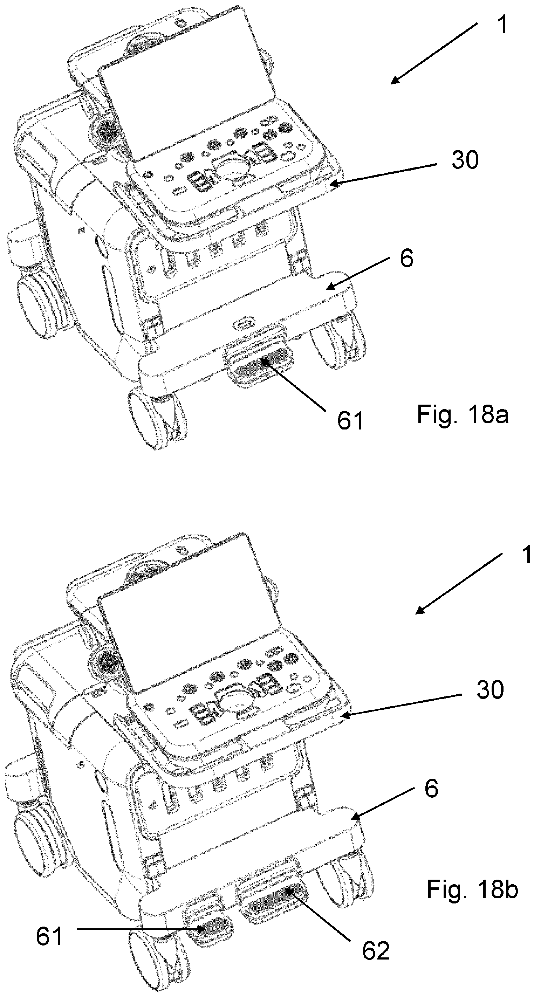

The system described above may be largely varied. For instance, Fig. 18a and 18b show perspective views of an ultrasound apparatus according to a further embodiment where the release button is a pedal 61 located on the wheel assembly 6 in addition to the pedal 62 use for the wheel braking system (if present). This configuration allows the space in the handle 30 (previously used for the button 31) to be left free for other purposes. The locking action on the control panel could also actuated by the same pedal of the wheel braking system to obtain a very compact design.

In a further variant, the button 31 could be surrounded by a LED strip 33 (shown in Fig. 18) that, thanks to a simple printed circuit board, is red coloured when the button 31 is released, and thus all the movements are locked, and green coloured when the button 31 is pushed and the movement of the pantograph 9 is allowed. This will provide a clear indication that the control panel 3 is in locked or unlocked state to increase safety.

As anticipated, in a still further variant, the pantograph and the described locking system could be applied to a trolley for portable apparatus (as illustrated in Fig. 20), where the upper bearing 92a is between the pantograph 9a and the console 3a supporting the portable ultrasound apparatus 1a, while the lower bearing 91a is, as usual, between the main body 5a and the pantograph 9a. The button 31a for the unlocking of the movements is located on the handle 30a of the console 3a. They could also be present the mechanical locks 7a.

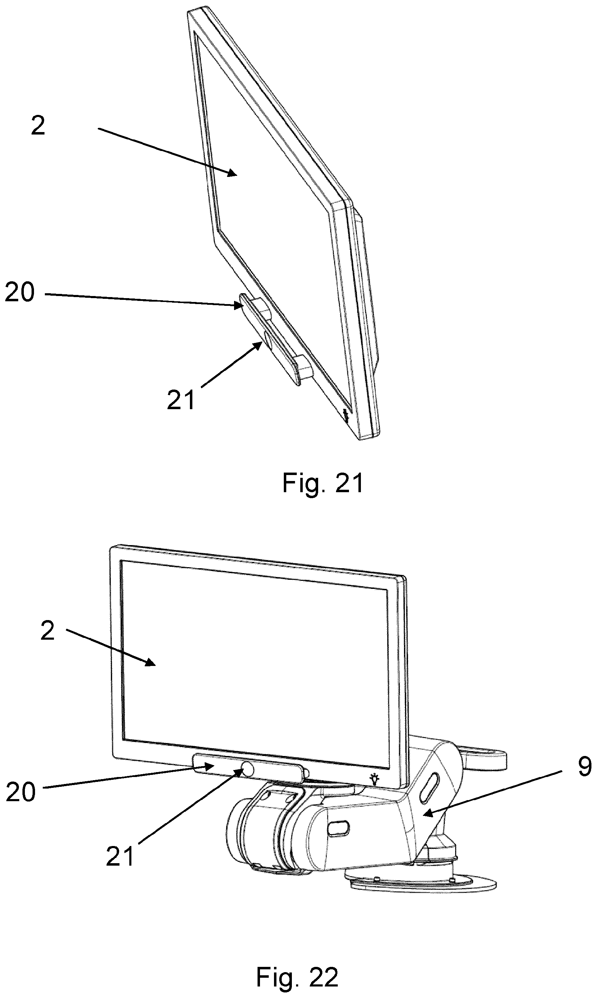

The last variant relates to a pantograph 9 which supports a monitor 2 with a handle 20 comprising the release button 21 and the lock/unlock system previously described (see Fig. 21, 22). In this case the pantograph 9 is slimmer than the one used for supporting the control panel because of the lighter weight. The handle 20 is used for positioning purpose of the monitor 2. Similarly, the mass moved by the electromagnet is smaller (at the limit, it could be the mass of the electromagnet itself).

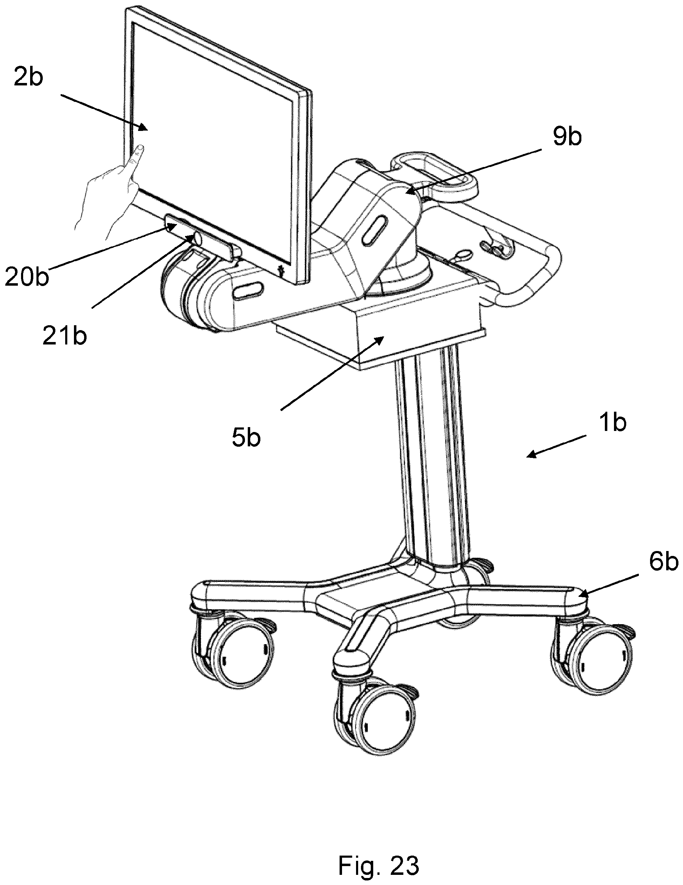

The same system could be used for a touch ultrasonic device, as shown in Fig. 23, where the LCD touch-control screen 2b (playing the role both of user interface and display) replaces the monitor 2. The handle 20b for the positioning of the LCD touch 2b comprises the button 21b, to unlock the pantograph 9b respect to the main body 5b of the trolley 1b.

Obviously, any combination of the previous variants is part of the present disclosure.

In conclusion, the system according to embodiments herein represents an improvement over the known systems as
- it can unlock at the same time all the degree of freedom, without lag and without a control unit;
- it allows a wide range of positioning, without intermediate step;
- it's a fully manual system, that allows the positioning in only one gesture;
- same parts (e.g. electromagnets) or subassemblies (e.g. mechanical lock) can be used overall for cost saving;
- it can be integrated with advantageous mechanical locks for a greater safety during transportation;
- the cleanability is guaranteed, as it has inner locks, without external elements (like protruding levers) that could retain dirt;
- it is noiseless and pleasant to use;
- as a consequence of the above points, it's simple, safe, convenient and of straightforward usage.

Another advantage resides in the fact that the main locks operate only for rotations, allowing, in case of an ultrasound system supplied by batteries and during the transportation (when the system is unplugged), the up and down movement in case of need, for example when a door must be crossed or the apparatus needs to enter in a lift.

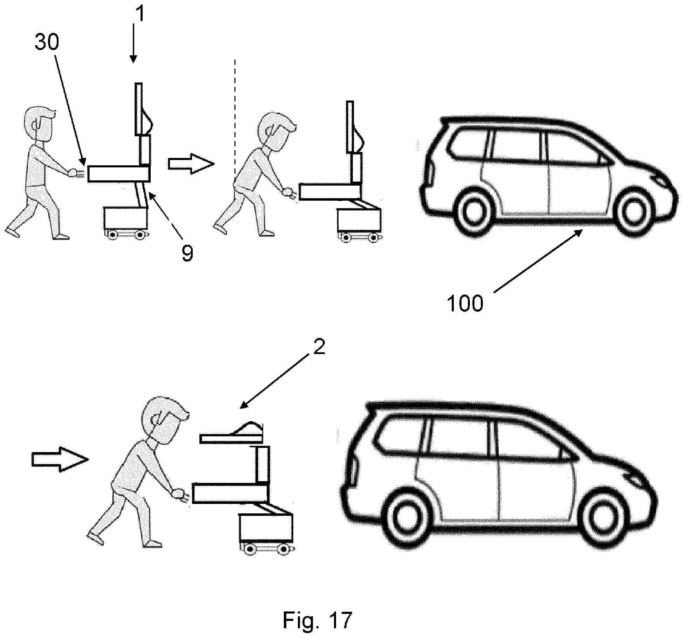

On the other hand, for the shipping from the manufacturer to the end user, the batteries are usually disconnected so the up and down movement of the pantograph 9 is always prevented. Similarly, as shown in Fig. 17, if the apparatus 1 needs to be transported by a MPV 100 (Multi-Purpose Vehicle) for mobile service, the transportation up to the vehicle 100 can be easily carried out with the pantograph 9 (and so the handle 30) in the high position, allowing the user to maintain a straight posture, while once arrived at the car 100 the pantograph 9 can be lowered for a more compact configuration.

## Claims

1. A cart-borne ultrasonic imaging system comprising:
a cart housing (5) mounted on wheels (6);
a control panel (3) or a support (3a) for a portable ultrasound unit (1a);
an articulating arm assembly (9) to which the control panel (3) is connected for adjusting the position of the control panel (3), or the support (3a) for a portable ultrasound unit (1a) is connected for adjusting the position of the support (3a) for a portable ultrasound unit (1a), the articulating arm assembly (9) including a first lower arm (109) rotatably mounted on the cart housing (5) and a second upper arm (209) pivotably connected to the first lower arm (109) and rotatably connected to the control panel (3), or the support (3a) for a portable ultrasound unit (1a), wherein at least one of the arms includes a 4-bar linkage optionally including a main damping member (97); and
a locking mechanism which acts to restrict motion of the articulating arm assembly (9), **characterised in that**
the locking mechanism comprises one or more electromagnetic elements (95, 96, 98) controllable to exert a magnetic force on movable actuating elements comprising:
a friction mass (950) coupled with a corresponding braking element (93, 94) acting to restrict rotation of the first lower arm (109) with respect to the cart (5) and/or of the control panel (3) / the support (3a) for a portable ultrasound unit (1a) with respect to the second arm (209); and/or
a locking member (981) kinematically coupled with the main damping member (97) acting to restrict the movement of such damping member (97) and thus of the 4-bar linkage,
wherein the electromagnetic elements (95, 96, 98) are electrically actuated by a switch (32) so that, upon activating the switch, the electromagnetic elements (95, 96, 98) are energized to move synchronously the actuating elements in an unlocking position.

2. The system according to claim 1, wherein at least one electromagnetic element is configured to move, when energized, the corresponding friction mass (950) from a locking position, wherein such mass is in contact with a braking element (93, 94) integral with the first arm (109) or the second arm (209), to an unlocking position, wherein such mass does not interfere with such braking element to restrict rotation of the first lower arm (109) with respect to the cart (5) and/or of the control panel (3) / the support (3a) for a portable ultrasound unit (1a) with respect to the second arm (209).

3. The system according to claim 1 or 2, wherein the locking mechanism is configured to restrict the rotation of the lower arm (109) with respect to the cart (5) and/or of the control panel (3) / support (3a) with respect to the upper arm (209) on a horizontal plane, wherein the braking element (93, 95) is an element having, at least partially, the shape of a disk integral with the lower arm (109) and/or the upper arm (209) and the electromagnetic element (95, 96) is housed on the cart (5) and/or the control panel (3) / support (3a) to exert a magnetic force to move the friction mass (950) in a direction transversal to the disk, particularly perpendicularly to the disk, to realize a magnetic-controllable disk brake.

4. The system according to any preceding claim, further comprising a rotary bearing (91, 92) between the lower arm (109) and the cart (5) and/or between the control panel (3) / support (3a) and the upper arm (109), wherein the braking element (93, 94) is an element having, at least partially, the shape of a disk integral with the part of the bearing associated with the lower arm and/or the upper arm.

5. The system according to any preceding claim, comprising:
a first electromagnetic element (95) coupled with a corresponding braking element (93) to lock/unlock rotational movement of the first arm (109) with respect to the cart (5);
a second electromagnetic element (96) coupled with a corresponding braking element (94) to lock/unlock rotational movement of the control panel (3) / support (3a) with respect to the second arm (209);
a third electromagnetic element (98) coupled with the main damping element (97) to lock/unlock the movement of the 4-bar linkage (98),
wherein the electromagnetic elements (95, 96, 98) are actuated synchronously by the same switch (32) so that, upon activating the switch (32), the full motion of the articulating arm assembly (9) is allowed.

6. The system according to any preceding claim, wherein the switch (32) is selected from the group consisting in: a pedal located on the cart, a button located on a frontal handle of the control panel or of the support for the portable ultrasound device, a button located on the cart, a button located on the frame of a flat panel display electrically coupled to imaging electronics contained in the cart housing and/or in the portable ultrasound unit, a pedal used to brake the wheels of the cart or combination thereof.

7. The system according to any preceding claim, wherein elastic elements are provided to keep the friction masses pushed onto the braking elements when the electromagnetic elements are not actuated by the switch.

8. The system according to any preceding claim, comprising an O-ring, a gasket, a rubber element or the like (951) on the surface of the friction elements facing the braking elements to increase friction.

9. The system according to any preceding claim, comprising one or more damping elements further to the main damping element (97) to balance the forces acting on the 4-bar linkage (98) when in unlocked position.

10. The system according to claim 9, wherein at least one of the one or more further damping elements is lockable/unlockable by the same or a different electromagnetic element (98) locking/unlocking the main damping element (97).

11. The system according to any preceding claim, further comprising an indicator (31) providing information on the status of the locking mechanism, particularly with a luminous indication in green/red or any other suitable colour, for example surrounding the button actuating the electromagnet(s).

12. The system according to any preceding claim, further comprising a mechanical lock (7) to secure the magnetic braking during transportation, such lock comprising a lever (71) moving a slidable pin (77) into a corresponding hole of the rotary bearing(s) when secure locking is to be achieved.

13. The system of claim 12 wherein the mechanical lock (7) further comprises a pin holder (72) and an elastic element (73), wherein the pin (77) is installed inside a cavity (721) of the pin holder (72) against the force of the elastic element (73) so that, when the lever (71) is activated, the pin (77) slides consequently inside the pin holder (72) to protrude from the pin holder (72) to engage a hole in a bracket (921, 911) integral with the corresponding bearing (92, 91), locking its rotation.

14. The system according to claim 12 or 13, wherein the lever (71) of the mechanical lock (7) is located on the cart (5) and/or on the control panel (3) / support (3a) for a portable ultrasound device in a position accessible by a user.

15. The system according to any claim 12 to 14, wherein the lever (71) is located in a position remote from the mechanical lock (7) accessible to a user and connected with the mechanical lock (7) through a wiring cable (78) to transmit motion from the lever (71) to the slidable pin (77).
